# FASCICULE DE BREVET EUROPEEN

(11) **EP 0 989 823 B1**
(45) Date de publication et mention de la délivrance du brevet: **19.05.2004**
(21) Numéro de dépôt: 98932215.1
(22) Date de dépôt: 16.06.1998
(51) Int. Cl.: A61B 17/04, A61B 17/062

(54) **APPLICATEUR POUR LA MISE EN PLACE D'UNE AIGUILLE CHIRURGICALE**
APPLIKATOR ZUM EINBRINGEN EINER CHIRURGISCHEN NADEL
APPLICATOR FOR SETTING A SURGICAL NEEDLE

(30) Priorité: 16.06.1997 FR 9707681
(43) Date de publication de la demande: 05.04.2000
(73) Titulaire: SOPRANE SA, F-69006 Lyon (FR)
(72) Inventeur: LOUBENS, Thierry, F-69003 Lyon (FR); RIOU, Lionel, F-69003 Lyon (FR); WATRELOT, Antoine, F-69008 Lyon (FR)
(74) Mandataire: Schmitt, John
(86) Numéro de dépôt international: PCT/FR1998/001263
(87) Numéro de publication internationale: WO 1998/057584

(56) Documents cités:
- EP-A- 0 529 675
- WO-A-92/05828
- US-A- 5 037 433
- US-A- 5 176 691
- US-A- 5 281 237

## Description

La présente invention est relative à un applicateur pour la mise en place d'une aiguille solidaire de son fil de suture dans le site chirurgical.

On connaît d'après le brevet WO 92/05828 un applicateur pour la mise en place d'une aiguille solidaire d'un fil de suture dans le site opératoire. L'applicateur comporte un manchon solidaire de moyens de préhension. A l'intérieur du manchon est introduite une canule creuse comportant à l'une de ses extrémités un mandrin déformable élastiquement pour la retenue d'une aiguille à l'intérieur d'un alésage tandis qu'à l'autre extrémité la canule est solidaire d'un moyen de préhension semblable à celui du manchon. L'aiguille est déformée à l'intérieur du manchon de manière à être contrainte dans une position sensiblement allongée tandis que son fils de suture traverse la canule pour venir déboucher au niveau des moyens de préhension.

On connaît également d'après le brevet US 5 037433 un dispositif pour la réalisation de sutures à l'aide d'une aiguille. Le dispositif comporte un tube dans lequel est introduite une canule et un dispositif pour la réalisation du noeud de suture. La canule comporte dans sa partie interne un premier alésage qui se prolonge par un autre alésage de diamètre inférieur au premier, afin de délimiter un épaulement. A l'intérieur de l'alésage est introduite une aiguille qui est disposée de manière que son fil sorte de la canule par l'alésage pour venir se nouer autour du dispositif. A l'intérieur de l'alésage est introduite une tige prévue d'un diamètre externe légèrement inférieur à celui interne de l'alésage. La tige est solidaire d'une autre tige dont le diamètre externe est légèrement inférieur à celui interne de l'alésage. L'aiguille est extraite de la canule par l'intermédiaire de la tige qui coulisse respectivement à l'intérieur des alésages. On constate que le dispositif décrit ne montre pas de moyens de retenue permettant de maintenir la tige à l'intérieur de la canule dans une position déterminée de manière que la tige soit en appui contre l'aiguille.

L'applicateur suivant la présente invention est destiné également aux procédures endoscopiques afin de coopérer, par exemple, avec un trocart de faible diamètre pour amener et libérer l'aiguille dans le site chirurgical.

L'applicateur conforme à l'invention comprend un manchon dans lequel est contrainte dans une position sensiblement allongée l'aiguille, une tige qui pénètre dans le manchon pour l'extraction de ladite aiguille et des moyens de retenue constitués d'une platine de forme oblongue, dont l'une des faces est solidaire de deux pinces qui coopèrent respectivement avec la tige et le manchon, lesdits moyens de retenue permettent, avant l'extraction de l'aiguille, de maintenir la tige à l'intérieur du manchon dans une position déterminée de manière que l'extrémité libre de ladite tige soit en appui contre l'aiguille et des moyens de tension constitués d'une bague souple qui est introduite dans un logement ménagé dans le manchon de manière à coincer légèrement le fil de suture entre la bague et la paroi interne dudit logement qui permettent pendant l'extraction de l'aiguille de maintenir à l'intérieur dudit manchon le fil de suture tendu.

L'applicateur suivant la présente invention comporte un manchon comprenant une tête cylindrique pourvue sur sa face externe d'une empreinte circulaire qui coopère avec la pince de la platine, tandis que la tige comprend une butée munie d'une empreinte autour de laquelle s'encliquette la pince de ladite platine de manière à maintenir une distance (L) constante entre ladite tête et ladite butée pour assurer que l'extrémité libre de la tige soit, d'une part en contact avec l'aiguille, et d'autre part à une distance (L') de l'extrémité libre du manchon.

L'applicateur suivant la présente invention comporte une distance (L') qui est identique à celle (L) prévue entre la butée de la tige et la tête du manchon.

L'applicateur suivant la présente invention comporte une platine qui est percée en son milieu d'un trou débouchant coopérant avec une fente qui débouche dans une gorge ménagée sur la périphérie de ladite platine afin de recevoir et d'enrouler le fil de suture se trouvant à l'extérieur de l'applicateur.

L'applicateur suivant la présente invention comprend un manchon qui est percé sur toute sa longueur d'un alésage dans lequel est introduit l'aiguille, tandis qu'à l'une de ses extrémités ledit manchon est solidaire d'une tête cylindrique pourvue d'un logement débouchant coaxialement dans l'alésage.

La description qui va suivre en regard des dessins annexés, donnés à titre d'exemples non limitatifs, permettra de mieux comprendre l'invention, les caractéristiques qu'elle présente et les avantages qu'elle est susceptible de procurer :
Figure 1 est une coupe illustrant l'applicateur pour la mise en place d'une aiguille suivant la présente invention.
Figures 2 et 3 sont des vues de côté montrant l'applicateur avant la mise en place des moyens de retenue de la tige par rapport au manchon.
Figure 4 est une vue de dessus représentant l'applicateur suivant la présente invention.
Figure 5 est une vue schématique de l'applicateur suivant la présente invention dans un trocart avant la libération de l'aiguille.
Figure 6 est une vue semblable à celle de figure 5, mais montrant l'aiguille dans le site chirurgical.
Figures 7 et 8 sont des vues illustrant une variante de la tige venant en appui contre l'aiguille maintenue dans l'applicateur.
Figures 9 à 11 sont des vues représentant une variante de l'applicateur permettant de l'utiliser comme pousse noeud.

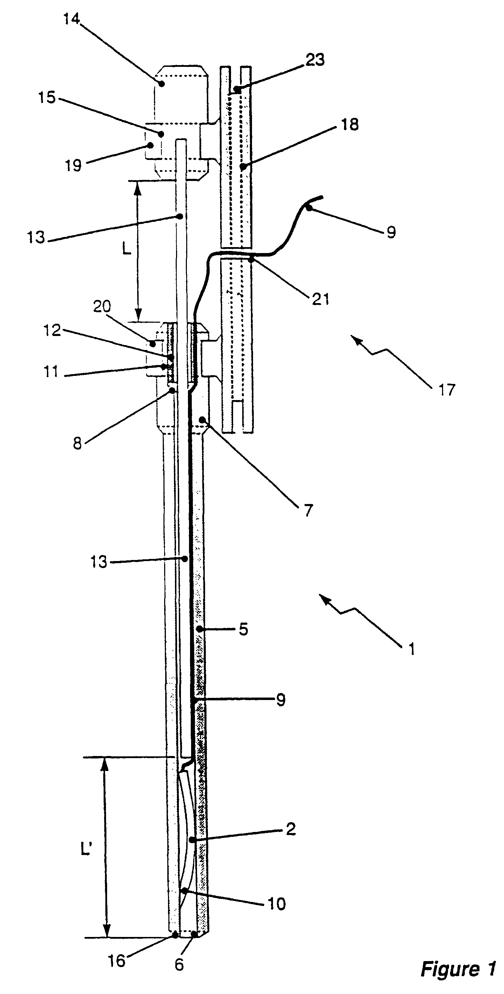

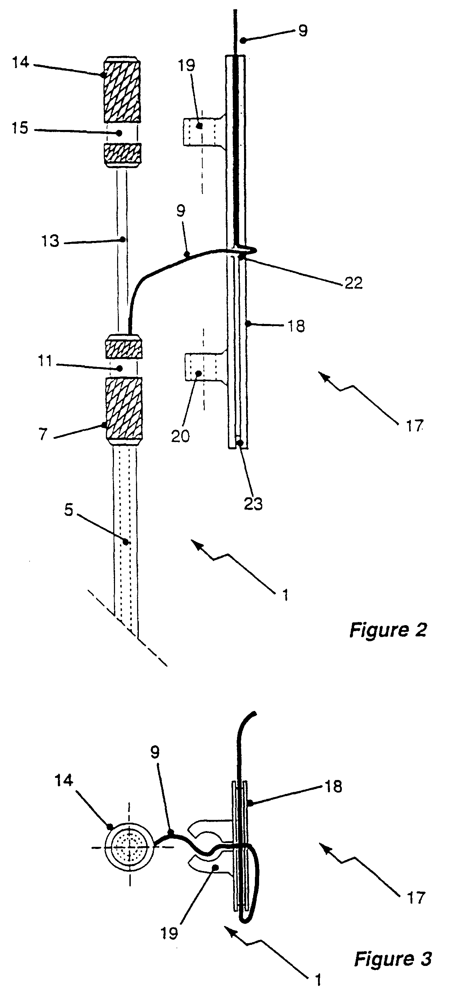

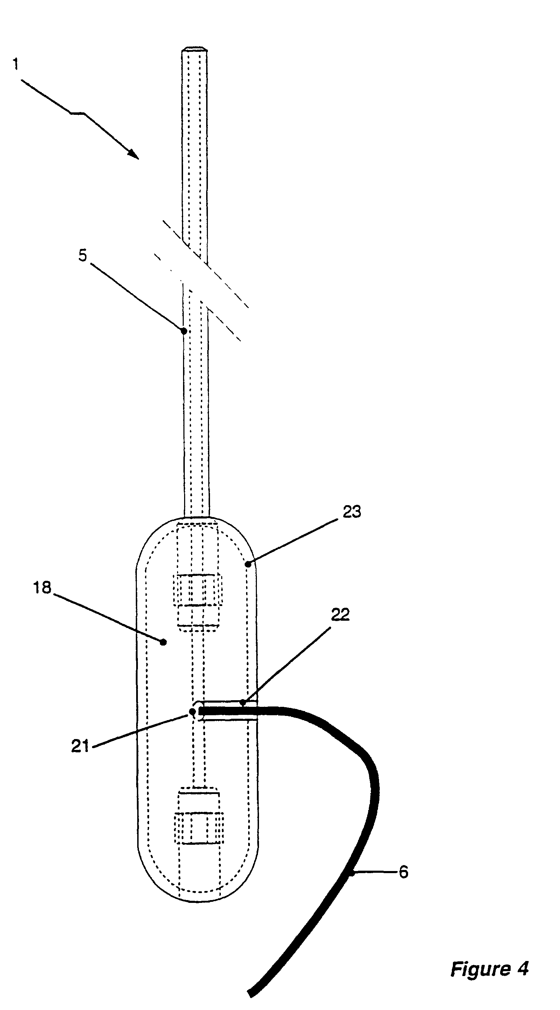

On a représenté en figures 1 à 4 un applicateur 1 permettant la mise en place d'une aiguille 2 solidaire d'un fil de suture 9 dans le site chirurgical par l'intermédiaire d'un trocart 3 qui a été préalablement placé au travers de la paroi 4 d'un patient.

L'applicateur 1 comprend un manchon cylindrique 5 percé sur toute sa longueur, et parallèlement à son axe longitudinal, d'un alésage 6 dans lequel sont logés l'aiguille 2 et son fil de suture 9.

Le manchon 5 comporte à l'une de ses extrémités une tête cylindrique 7 qui est prévue d'un diamètre supérieur à celui du reste du corps dudit manchon. A l'intérieur de la tête 7 est percé, coaxialement à l'alésage 6, un logement 8 qui est d'un diamètre supérieur à celui dudit alésage.

Le logement 8 communique avec l'alésage 6 de manière que le fil de suture 9 solidaire de l'aiguille 2 puisse sortir en direction de l'extérieur du manchon. En effet, l'aiguille 2 est disposée dans l'alésage 6 du manchon 5 de manière que sa pointe 10 soit dirigée en direction de l'extrémité libre 16 et opposée à celle portant la tête 7.

On note que la pointe 10 de l'aiguille 2 se trouve légèrement décalée par rapport à l'extrémité libre 16 du manchon 5.

La tête 7 du manchon 5 comprend sur sa face externe une empreinte circulaire 11 dont on verra mieux plus loin la fonction.

Dans le logement 8, est introduit coaxialement une bague souple 12 qui constitue des moyens de tension du fil de suture 9 à l'intérieur de l'alésage 6 du manchon 5. En effet, la bague vient légèrement coincer le fil de suture 9 contre la paroi interne du logement 8. Dans ces conditions, on remarque que le fil de suture 9 est disposé entre la face externe de la bague 12 et la paroi interne du logement 8.

L'aiguille 2 est légèrement retenue, du fait de sa déformation élastique, dans l'alésage 6 du manchon 5 permettant, lorsqu'une traction est exercée sur le fil de suture 9, de le tendre entre l'aiguille et la bague 12 à l'intérieur de l'alésage 6.

On note que la pointe 10 de l'aiguille 2 se trouve légèrement décalée par rapport à l'extrémité libre du manchon 5 se trouvant à l'opposé de la tête 7.

L'applicateur 1 comprend une tige 13 qui est solidaire à l'une de ses extrémités d'une butée cylindrique 14 comportant sur sa face externe une empreinte circulaire 15 dont on verra mieux plus loin la fonction.

La tige 13 est introduite dans le manchon 5 de manière à venir en appui contre l'aiguille 2. La tige 13 traverse la bague 12 et l'alésage 6, de manière que le fil de suture 9 soit disposé entre ladite tige et la paroi dudit alésage.

Des moyens de retenue 17 permettent de maintenir la tige 13 par rapport au manchon 5, de manière que l'extrémité libre de la tige 13 opposée à la butée 14 soit toujours en appuie contre l'aiguille 2.

Les moyens de retenue 17 consistent en une platine 18 solidaire sur l'une de ses faces de deux pinces 19 et 20, qui sont déformées élastiquement pour constituer deux clips de fixation. Chaque pince 19 et 20 coopère respectivement avec l'empreinte 15 de la butée 14 solidaire de la tige 13, et l'empreinte 11 de la tête 7 du manchon 5.

La position des deux pinces 19 et 20 sur la platine 18 permet de maintenir entre la butée 14 de la tige 13 et la tête 7 du manchon 5 une distance constante L. Cette distance L assure que l'extrémité libre de la tige 13, c'est à dire celle opposée à la butée 14, soit d'une part en contact avec l'aiguille 2, et d'autre part à une distance L', identique à celle L de l'extrémité libre 16 du manchon 5.

La platine 18 constitue également une bobine pour l'enroulement du fil 9 à l'extérieur de l'applicateur 1.

La platine 18 présente une forme allongée sensiblement oblongue qui est percée en son milieu d'un trou débouchant 21.

Le trou 21 communique par l'intermédiaire d'une fente 22 avec une gorge 23 qui est ménagée sur toute la périphérie de la platine 18. La fente 22 est prévue sur la face opposée à celle recevant les pinces 19 et 20.

La partie du fil de suture 9 se trouvant à l'extérieur du manchon 5 traverse le trou central 21 pour venir, par l'intermédiaire de la fente 22, s'enrouler dans la gorge périphérique 23. L'extrémité libre du fil 9 peut être, par exemple coincée dans la gorge au moyen d'un petit bouchon en silicone, non représenté, permettant la retenue du fil de suture sur la platine 18.

La préparation de l'applicateur 1 en salle blanche ou stérile consiste :
- A introduire une aiguille 2 susceptible de se déformer élastiquement ou ayant des caractéristiques analogues, qui est solidaire d'un fil de suture 9 dans l'alésage 6 du manchon 5 de manière que sa pointe 10 soit dirigée du côté de l'extrémité libre 16,
- A disposer la bague de retenue 12 dans le logement 8 du manchon 5 pour coincer légèrement le fil de suture 9 entre la bague et la paroi du logement,
- A tirer légèrement sur le fil de suture 9 afin de le tendre à l'intérieur de l'alésage 6 du manchon 5,
- A introduire la tige 13 dans l'alésage 6 du manchon 5 pour placer le fil de suture 9 entre ladite tige et la paroi interne dudit alésage,
- A bloquer la tige 13 par rapport au manchon 5 au moyen de la platine 18 pour que la butée 14 se trouve à une distance L de la tête 7 assurant que l'extrémité de ladite tige soit en contact avec l'aiguille 2,
- A enrouler le fil de suture 9 autour de la platine 18,
- A fixer le fil de suture sur la platine 18,
- A placer l'applicateur 1 muni de l'aiguille 2 dans un sachet stérile.

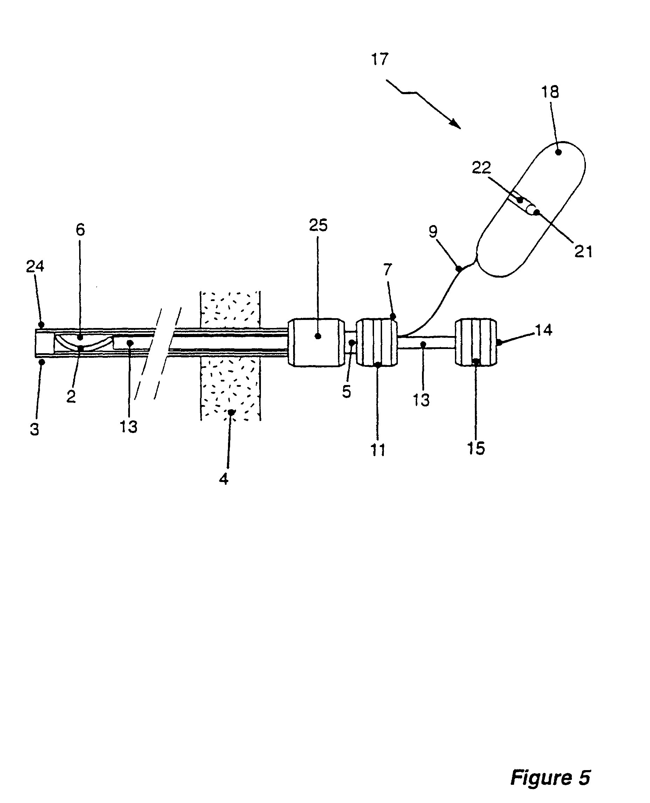

En figure 5 on a montré l'applicateur 1 disposé à l'intérieur du trocart 3 qui a été préalablement placé au travers de la paroi 4 d'un patient.

Le trocart est constitué d'un tube 24 solidaire à l'une de ses extrémités d'une jupe circulaire 25 servant de butée à l'applicateur lors de la mise en place de l'aiguille 2 dans le site chirurgical comme on le verra mieux plus loin.

Le tube 24 du trocart 3 est prévu pour recevoir le manchon 5 de l'applicateur 1, tandis que la tête 7 vient en appuie contre la jupe circulaire 25.

Le chirurgien procède ensuite au retrait des moyens de retenue 17, et plus particulièrement de la platine 18 pour libérer la tige 13 par rapport au manchon 5.

On note que la partie du fil de suture 9 se trouvant à l'extérieur de l'applicateur 1 peut rester enroulée autour de la bobine ou platine 18 pendant l'opération d'introduction de l'aiguille 2 dans le site chirurgical.

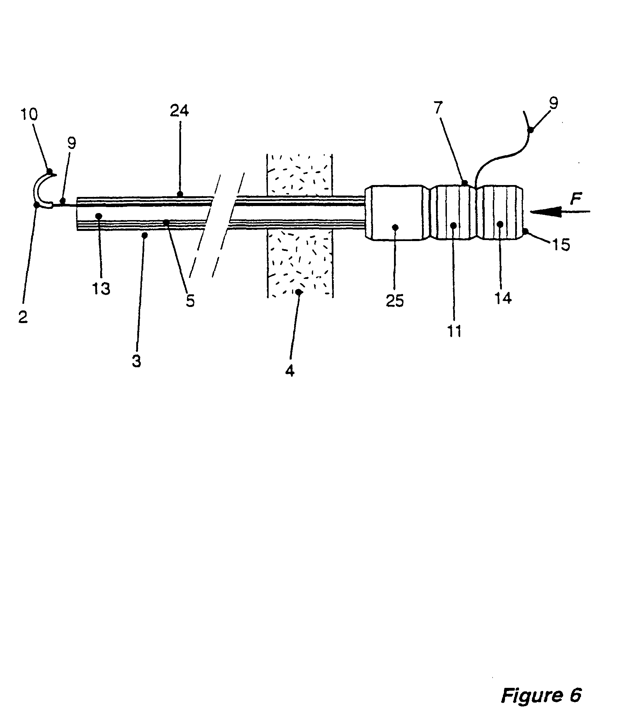

En figure 6 on a montré l'extraction de l'aiguille 2 du manchon 5 de l'applicateur 1.

En effet, il suffit au chirurgien d'appuyer, suivant la flèche F, sur la butée 14 de la tige 13, pour pousser par l'intermédiaire de ladite tige 13, l'aiguille 2 à l'extérieur du manchon 5. Le déplacement de la tige 13 est limité dans sa course jusqu'à ce que la butée 14 soit en contact avec la tête 7 du manchon 5.

On note que le déplacement de la butée 14 d'une distance L garantie que l'extrémité libre de la tige 13 se soit déplacée d'une distance identique L' et que l'aiguille 2 a été extraite du manchon 5.
De plus, les moyens de tension disposés dans la tête 7, et constitués par la bague 12, permettent, lors de l'extraction de l'aiguille 2, que le fil de suture 9 reste tendu à l'intérieur de l'alésage 6 pour éviter toute possibilité de bourrage de ce dernier à l'intérieur du manchon 5 et autour de la tige 13.

On constate que l'aiguille 2 du fait de son élasticité reprend sa forme en arc de cercle.

En outre, l'aiguille 2 est retenue au moyen de son fil de suture 9 au voisinage du trocart 3 par l'intermédiaire du fil de suture 9 qui est, soit légèrement coincé par la bague 12, soit simultanément retenu par la bague 12 et la platine 18.

Ensuite, le chirurgien récupère l'aiguille 2 au moyen d'une pince, non représentée, en retirant conjointement la tige 13 du manchon 5.

Préalablement à la récupération de l'aiguille 2 dans le site chirurgical, le fil de suture est entièrement déroulé de la bobine pour permettre au chirurgien son intervention.

Pour facilité la préhension du manchon 5, il est possible de rajouter autour de la tête 7 des moyens de blocage, non représentés, des doigts du chirurgien pour qu'il puisse utiliser l'applicateur d'une seule main comme une seringue.

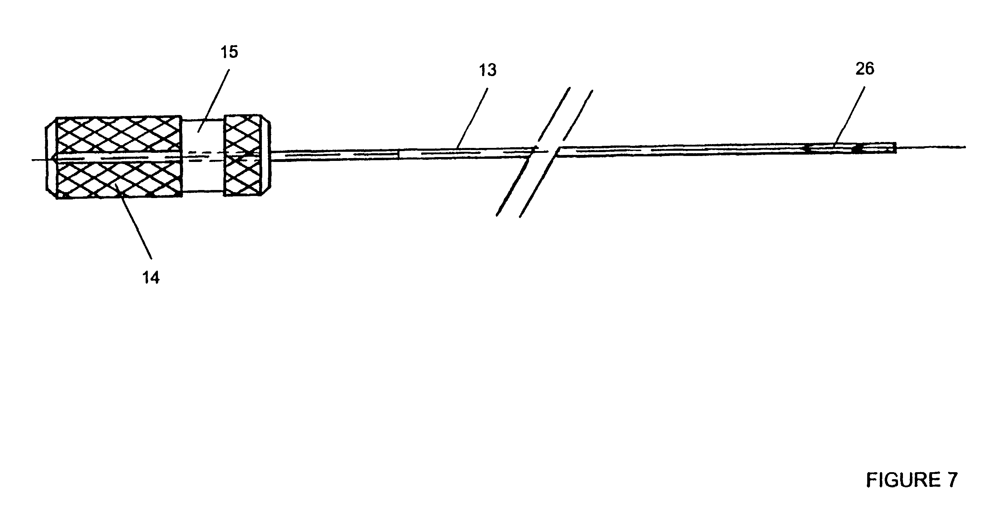

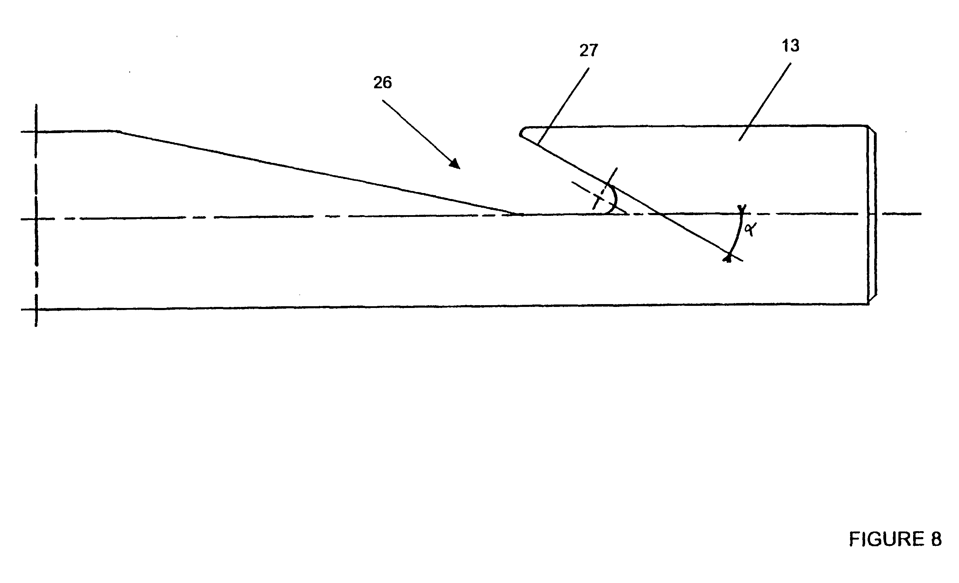

En figures 7 et 8 on a représenté une variante de la tige 13 introduite dans l'applicateur 1 suivant la présente invention.

La tige 13 comprend à son extrémité libre opposée à la butée cylindrique 14, une encoche 26 en forme de crochet tourné en direction de ladite butée. L'encoche 26 ménagée dans l'épaisseur de la tige 13 présente un pan 27 qui est incliné d'un angle α d'environ 30 degrés.

L'inclinaison du pan 27 délimite l'ouverture de l'encoche 26. On note que la profondeur de l'encoche 26 ne dépasse pas l'axe central et longitudinal de la tige 13.

La tige 13 modifiée permet, d'une part de réaliser la même fonction décrite précédemment afin d'introduire l'aiguille 2 dans le site opératoire, et d'autre part de récupérer à l'aide du crochet 26 le fil de suture 9 au milieu du site opératoire, et de le sortir à l'extérieur du corps humain en laissant l'aiguille à l'intérieur pour réaliser des noeuds extra-corporels.

L'extraction du fil de suture 9 est réalisée, soit au travers de l'applicateur 1, soit au travers du trocart 3 selon les desiderata du chirurgien.

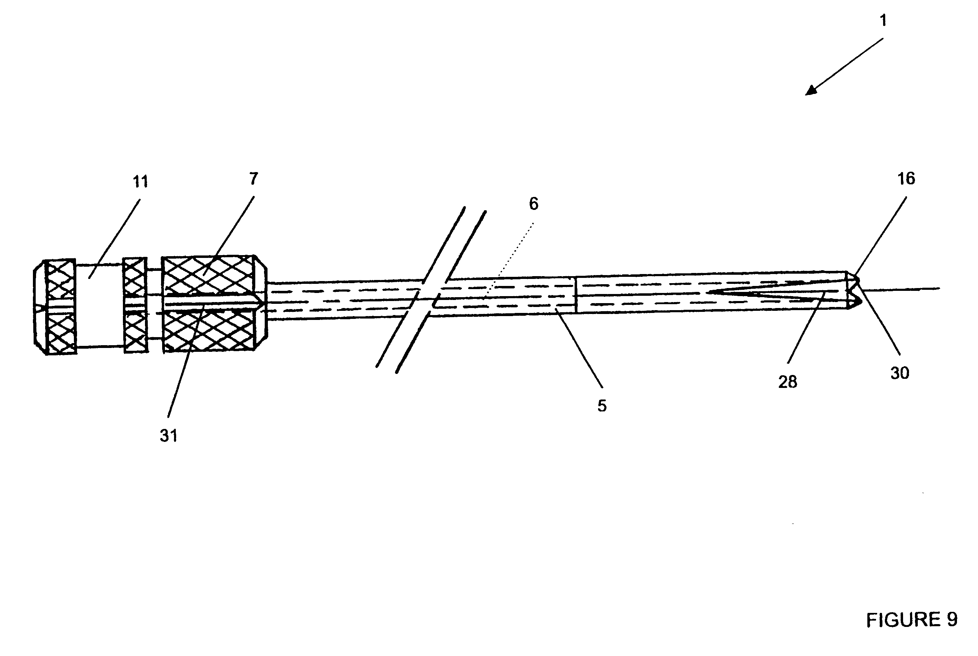

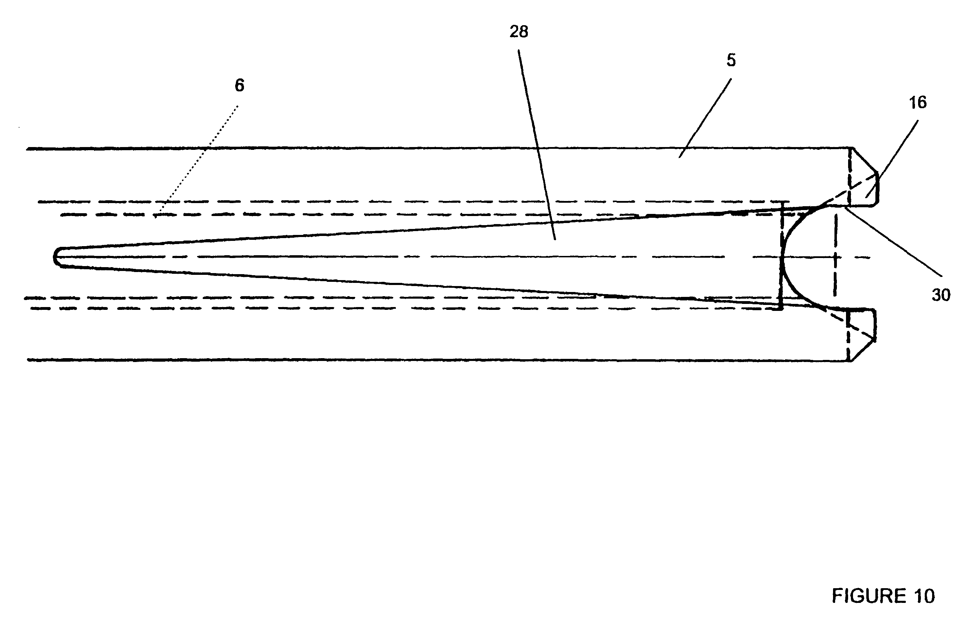

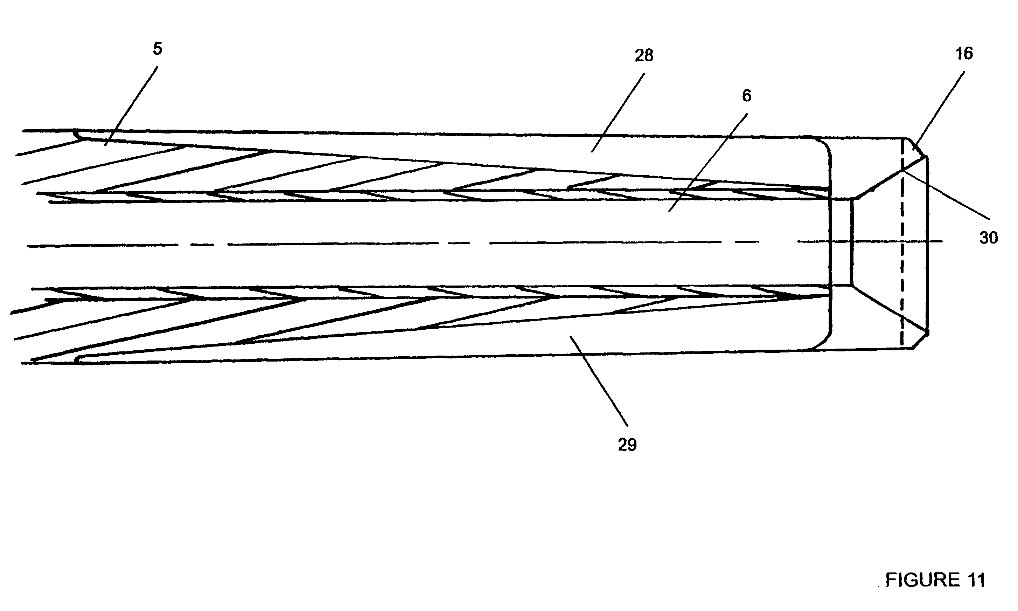

En figures 9 à 11 on a montré une variante de l'applicateur 1, et plus particulièrement du manchon 5, qui comprend à son extrémité libre 16 et opposée à la tête 7, des moyens de guidage du fil de suture 9 lorsque les noeuds sont réalisés à l'extérieur du corps humain ou extra-corporel.

Le manchon 5 comprend à partir de l'extrémité 16, et en direction de la tête 7, deux rainures 28 et 29 diamétralement opposées et parallèles à l'axe longitudinal de l'applicateur 1.

Chaque rainure 28 et 29 présente un profil en forme de V dont la base la plus ouverte, ou la plus large, est tournée du côté de l'extrémité 16. Egalement, chaque rainure 28 et 29 présente une profondeur décroissante le long de la périphérie du manchon 5, afin que le niveau le plus profond soit du côté de l'extrémité libre 16 et de la base la plus ouverte du V.

L'extrémité 16 du manchon 5 comporte une fente 30 disposée perpendiculairement aux rainures 28, 29 de manière que chaque rainure coopère avec ladite fente. Le diamètre de la fente 30 est sensiblement identique aux dimensions de la base la plus ouverte du V formant le profil de chaque rainure 28, 29. La fente 30 forme une creusure à l'intérieur du manchon 5 qui communique avec l'alésage interne 6 dans lequel coulisse la tige 13 et l'aiguille 2 solidaire du fil de suture 9.

Enfin, la tête 7 du manchon 5 comporte respectivement dans l'alignement des rainures 28, 29, une empreinte 31 formant un repère au chirurgien pour positionner la fente 30, et les rainures dans le site opératoire.

En effet, le manchon 5 muni des rainures 28, 29, de la fente 30 et des empreintes 31, permet au chirurgien de réaliser des noeuds sur le fil de suture 9 à l'extérieur du corps humain. Le manchon 5 permet au chirurgien de placer le noeud dans la fente 31, tandis que chaque extrémité du fil de suture 9 est disposée dans les rainures 28 et 29 dudit manchon.

Le chirurgien introduit le manchon 5 à l'intérieur du trocart 3 en maintenant de part et d'autre dudit manchon les extrémités du fil de suture 9, afin de pousser et de faire glisser le noeud retenu dans la fente 31 dans le trocart pour l'amener à l'intérieur du site opératoire et ligaturer la plaie.

## Revendications

1. Applicateur pour la mise en place d'une aiguille solidaire d'un fil de suture dans un site chirurgical, comprenant un manchon (5) dans lequel est contraint dans une position sensiblement allongée l'aiguille (2), une tige (13) qui pénètre dans le manchon (5) pour l'extraction de ladite aiguille **caractérisé en ce qu**'il comprend en outre des moyens de retenue (17) constitués d'une platine (18) de forme oblongue, dont l'une des faces est solidaire de deux pinces (19, 20) qui coopèrent respectivement avec la tige (13) et le manchon (5), lesdits moyens de retenue (17) permettant, avant l'extraction de l'aiguille (2), de maintenir la tige (13) à l'intérieur du manchon (5) dans une position déterminée de manière que l'extrémité libre de ladite tige soit en appui contre l'aiguille (2) et des moyens de tension (12) constitués d'une bague souple qui est introduite dans un logement (8) ménagé dans le manchon (5) de manière à coincer légèrement le fil de suture (9) entre la bague et la paroi interne dudit logement permettant pendant l'extraction de l'aiguille (2) de maintenir à l'intérieur dudit manchon le fil de suture (9) tendu.

2. Applicateur suivant la revendication 1 **caractérisé en ce que** le manchon (5) comporte une tête cylindrique (7) pourvue sur sa face externe d'une empreinte circulaire (11) qui coopère avec la première pince (20) de la platine (18), tandis que la tige (13) comprend une butée (14) munie d'une empreinte (15) autour de laquelle s'encliquette la deuxième pince (19) de ladite platine (18) de manière à maintenir une première distance (L) constante entre ladite tête (7) et ladite butée (14) pour assurer que l'extrémité libre de la tige (13) soit, d'une part en contact avec l'aiguille (2), et d'autre part à une deuxième distance (L') de l'extrémité libre (16) du manchon (5).

3. Applicateur suivant la revendication 2 **caractérisé en ce que** ladite deuxième distance (L') est identique à celle (L) prévue entre la butée (14) de la tige (13) et la tête (7) du manchon (5).

4. Applicateur suivant la revendication 1 **caractérisé en ce que** la platine (18) est percée en son milieu d'un trou débouchant (21) coopérant avec une fente (22) qui débouche dans une gorge (23) ménagée sur la périphérie de ladite platine afin de recevoir et d'enrouler le fil de suture (9) se trouvant à l'extérieur de l'applicateur (1).

5. Applicateur suivant la revendication 1 **caractérisé en ce que** le manchon (5) est percé sur toute sa longueur d'un alésage (6) dans lequel est introduit l'aiguille (2), tandis qu'à l'une de ses extrémités ledit manchon est solidaire d'une tête cylindrique (7) pourvue d'un logement (8) débouchant coaxialement dans l'alésage (6).

6. Applicateur suivant la revendication 5 **caractérisé en ce que** le manchon (5) est assujetti au niveau de la tête (7) de moyens de préhension permettant au chirurgien d'utiliser l'applicateur (1) comme une seringue.

7. Applicateur suivant la revendication 2 **caractérisé en ce que** la tige (13) comprend à son extrémité libre et opposée à la butée (14) une encoche (26) en forme de crochet.

8. Applicateur suivant la revendication 7 **caractérisé en ce que** l'encoche (26) présente un pan (27) qui est incliné d'un angle (α) de 30 degrés délimitant l'ouverture de ladite encoche.

9. Applicateur suivant la revendication 7 **caractérisé en ce que** la profondeur de l'encoche (26) ne dépasse pas l'axe central et longitudinal de la tige (13).

10. Applicateur suivant la revendication 1 **caractérisé en ce que** le manchon (5) comprend au niveau de son extrémité libre (16) deux rainures (28, 29) diamétralement opposées et parallèles à l'axe longitudinal dudit manchon et une fente (30) disposée perpendiculairement aux dites rainures.

11. Applicateur suivant la revendication 10 **caractérisé en ce que** chaque rainure (28, 29) présente un profil en V dont la base la plus large est tournée du coté de l'extrémité libre (16) dudit manchon.

12. Applicateur suivant la revendication 10 **caractérisé en ce que** chaque rainure (28, 29) présente une profondeur décroissante, de manière que le niveau le plus profond soit du coté de l'extrémité libre (16) dudit manchon.

13. Applicateur suivant la revendication 11 **caractérisé en ce que** le diamètre de la fente (30) est sensiblement identique aux dimensions de la base la plus ouverte du V formant le profil de chaque rainure (28, 29).

14. Applicateur suivant la revendication 10 **caractérisé en ce que** la fente (30) forme une creusure à l'intérieur du manchon (5) qui communique avec l'alésage interne (6) dudit manchon dans lequel coulisse la tige (13).

15. Applicateur suivant la revendication 10 **caractérisé en ce que** la tête (7) du manchon (5) comporte des empreintes (31) disposées respectivement dans l'alignement des rainures (28, 29).

## Patentansprüche

1. Applikator zur Platzierung einer mit einem Nähfaden fest verbundenen Nadel an einem chirurgischen Platz mit einem Rohrstück (5), in dem die Nadel (2) in einer im Wesentlichen ausgestreckten Position eingezwängt ist, und einem in das Rohrstück (5) eindringenden Schaft (13) für das Herausziehen der Nadel, **dadurch gekennzeichnet, dass** er außerdem Rückhaltemittel (17) umfasst, die von einer Platte (18) von länglicher Form gebildet werden, deren eine Seite fest mit zwei Klemmen (19, 20) verbunden ist, die jeweils mit dem Schaft (13) und dem Rohrstück (5) zusammenwirken, wobei die Rückhaltemittel (17) es vor dem Herausziehen der Nadel (2) gestatten, den Schaft (13) im Innern des Rohrstücks (5) in einer bestimmten Position zu halten, so dass das freie Ende des Schafts an der Nadel (2) anliegt, und Spannmittel (12), die von einem flexiblen Ring gebildet werden, der in eine Aufnahme (8) eingeführt wird, die in dem Rohrstück (5) ausgebildet ist, um den Nähfaden (9) zwischen dem Ring und der Innenwand der Aufnahme geringfügig einzuklemmen, wodurch es gestattet wird, während des Herausziehens der Nadel (2) den Nähdraht (9) im Innern des Rohrstücks gespannt zu halten.

2. Applikator nach Anspruch 1, **dadurch gekennzeichnet, dass** das Rohrstück (5) einen zylindrischen Kopf (7) umfasst, der auf seiner Außenseite mit einer kreisförmigen Vertiefung (11) versehen ist, die mit der ersten Klammer (20) der Platte (18) zusammenwirkt, während der Schaft (13) einen Anschlag (14) umfasst, der mit einer Vertiefung (15) versehen ist, in die die zweite Klammer (19) der Platte (18) einrastet, um einen ersten konstanten Abstand (L) zwischen dem Kopf (7) und dem Anschlag (14) aufrechtzuerhalten, um zu gewährleisten, dass das freie Ende des Schafts (13) einerseits Kontakt mit der Nadel (2) hat und andererseits sich in einem zweiten Abstand (L') von dem freien Ende (16) des Rohrstücks (5) befindet.

3. Applikator nach Anspruch 2, **dadurch gekennzeichnet, dass** der zweite Abstand (L') identisch zu demjenigen (L) ist, der zwischen dem Anschlag (14) des Schafts (13) und dem Kopf (7) des Rohrstücks (5) vorgesehen ist.

4. Applikator nach Anspruch 1, **dadurch gekennzeichnet, dass** die Platte (18) in ihrer Mitte von einem Mündungsloch (21) durchbohrt ist, das mit einem Schlitz (22) zusammenwirkt, der in eine Nut (23) mündet, die auf der Peripherie der Platte ausgebildet ist, um den außerhalb des Applikators (1) befindlichen Nähfaden (9) aufzunehmen und aufzuwickeln.

5. Applikator nach Anspruch 1, **dadurch gekennzeichnet, dass** das Rohrstück (5) auf seiner ganzen Länge von einer Bohrung (6) durchbohrt ist, in die die Nadel (2) eingeführt wird, während das Rohrstück an einem seiner Enden fest mit einem zylindrischen Kopf (7) verbunden ist, der mit einer Aufnahme (8) versehen ist, die koaxial in die Bohrung (6) mündet.

6. Applikator nach Anspruch 5, **dadurch gekennzeichnet, dass** an das Rohrstück (5) auf Höhe des Kopfs (7) Greifmittel angefügt sind, die es dem Chirurgen gestatten, den Applikator (1) wie eine Spritze zu halten.

7. Applikator nach Anspruch 2, **dadurch gekennzeichnet, dass** der Schaft (13) an seinem freien Ende gegenüber dem Anschlag (14) einen hakenförmigen Einschnitt (26) umfasst.

8. Applikator nach Anspruch 7, **dadurch gekennzeichnet, dass** der Einschnitt (26) eine Seitenfläche (27) aufweist, die um einen die Öffnung des Einschnitts begrenzende Winkel (α) von 30 Grad geneigt ist.

9. Applikator nach Anspruch 7, **dadurch gekennzeichnet, dass** die Tiefe des Einschnitts (26) nicht über die Mittel- und Längsachse des Schafts (13) hinausgeht.

10. Applikator nach Anspruch 1, **dadurch gekennzeichnet, dass** das Rohrstück (5) auf Höhe seines freien Endes (16) zwei diametral gegenüberliegende und zur Längsachse des Rohrstücks parallele Rillen (28, 29) und einen Schlitz (30) aufweist, der senkrecht zu den Rillen angeordnet ist.

11. Applikator nach Anspruch 10, **dadurch gekennzeichnet, dass** jede Rille (28, 29) ein V-förmiges Profil aufweist, dessen breiteste Basis zu der Seite des freien Endes (16) des Rohrstücks gewendet ist.

12. Applikator nach Anspruch 10, **dadurch gekennzeichnet, dass** jede Rille (28, 29) eine abnehmende Tiefe aufweist, so dass das tiefste Niveau sich auf der Seite des freien Endes (16) des Rohrstücks befindet.

13. Applikator nach Anspruch 11, **dadurch gekennzeichnet, dass** der Durchmesser des Schlitzes (30) im Wesentlichen identisch zu den Abmessungen der am weitesten offenen Basis des V's ist, das das Profil jeder Rille (28, 29) bildet.

14. Applikator nach Anspruch 10, **dadurch gekennzeichnet, dass** der Schlitz (30) im Inneren des Rohrstücks (5) eine Einsenkung bildet, die mit der Innenbohrung (6) des Rohrstücks in Verbindung steht, in der der Schaft (13) gleitet.

15. Applikator nach Anspruch 10, **dadurch gekennzeichnet, dass** der Kopf (7) des Rohrstücks (5) Vertiefungen (31) umfasst, die jeweils zu den Rillen (28, 29) ausgerichtet angeordnet sind.

## Claims

1. Applicator for setting a needle, secured to a suture thread, in place in a surgical site, comprising a sleeve (5) in which the needle (2) is held in an approximately elongate position, a rod (13) which enters the sleeve (5) to extract the said needle, **characterized in that** it additionally comprises retaining means (17) consisting of a plate (18) of oblong shape, one of the faces of which is secured to two grippers (19, 20), one of which collaborates with the rod (13) and the other of which collaborates with the sleeve (5), the said retaining means (17), before the needle (2) is extracted, allowing the rod (13) to be held inside the sleeve (5) in a position that is determined such that the free end of the said rod rests against the needle (2), and tensioning means (12), consisting of a flexible ring which is introduced into a housing (8) formed in the sleeve (5) so as to wedge the suture thread (9) slightly between the ring and the internal wall of the said housing, allowing the suture thread (9) to be kept taut inside the said sleeve while the needle (2) is being extracted.

2. Applicator according to Claim 1, **characterized in that** the sleeve (5) has a cylindrical head (7) provided on its external face with a circular recess (11) which collaborates with the first gripper (20) of the plate (18), while the rod (13) comprises a stop (14) equipped with a recess (15) around which the second gripper (19) of the said plate (18) clips so as to maintain a constant first distance (L) between the said head (7) and the said stop (14) to ensure that the free end of the rod (13) is, on the one hand, in contact with the needle (2) and, on the other hand, a second distance (L') away from the free end (16) of the sleeve (5).

3. Applicator according to Claim 2, **characterized in that** the said second distance (L') is identical to the distance (L) between the stop (14) of the rod (13) and the head (7) of the sleeve (5).

4. Applicator according to Claim 1, **characterized in that** the plate (18) is pierced at its middle with an emergent hole (21) collaborating with a slit (22) which opens into a groove (23) formed on the periphery of the said plate in which the suture thread (9) which is outside the applicator (1) can be wound and housed.

5. Applicator according to Claim 1, **characterized in that** the sleeve (5) is pierced, along its entire length, with a bore (6) in which the needle (2) is inserted, while, at one of its ends, the said sleeve is secured to a cylindrical head (7) which has a housing (8) opening coaxially into the bore (6).

6. Applicator according to Claim 5, **characterized in that** the sleeve (5) is fastened, in the region of the head (7), to means for holding which allow the surgeon to use the applicator (1) in the manner of a syringe.

7. Applicator according to Claim 2, **characterized in that** the rod (13) comprises, at its free end, which is the opposite end to the stop (14), a hook-shaped notch (26).

8. Applicator according to Claim 7, **characterized in that** the notch (26) has a surface (27) which is inclined by an angle (α) of 30 degrees delimiting the opening of the said notch.

9. Applicator according to Claim 7, **characterized in that** the depth of the notch (26) does not go beyond the central and longitudinal axis of the rod (13).

10. Applicator according to Claim 1, **characterized in that** the sleeve (5) has, at its free end (16), two channels (28, 29) which are diametrically opposed and parallel to the longitudinal axis of the said sleeve and a slit (30) arranged at right angles to the said channels.

11. Applicator according to Claim 10, **characterized in that** each channel (28, 29) has a V-shaped profile, the widest end of which faces towards the free end (16) of the said sleeve.

12. Applicator according to Claim 10, **characterized in that** each channel (28, 29) has a decreasing depth, so that the deepest point is at the free end (16) of the said sleeve.

13. Applicator according to Claim 11, **characterized in that** the diameter of the slit (30) is approximately identical to the dimensions of the most open end of the V which forms the profile of each channel (28, 29).

14. Applicator according to Claim 10, **characterized in that** the slit (30) forms a hollow inside the sleeve (5), which communicates with the internal bore (6) of the said sleeve in which the rod (13) slides.

15. Applicator according to Claim 10, **characterized in that** the head (7) of the sleeve (5) has recesses (31) each arranged in line with one of the channels (28, 29).
